# EUROPEAN PATENT APPLICATION

(11) **EP 2 940 205 A1**
(43) Date of publication of application: **04.11.2015**
(21) Application number: 13868398.2
(22) Date of filing: 06.12.2013
(51) Int. Cl.: D06M 11/71, A61C 17/00, B01J 20/04, B01J 20/30, D06M 23/08

(54) **FUNCTIONAL NONWOVEN FABRIC AND MANUFACTURING METHOD THEREFOR**

(30) Priority: 28.12.2012 JP 2012286823
(71) Applicant: JAPAN VILENE COMPANY, LTD., Tokyo 104-8423 (JP); SofSera Corporation, Tokyo 160-0022 (JP)
(72) Inventor: MATSUBAYASHI, Yasuko, Koga-shi Ibaraki 306-0213 (JP); KATO, Koichi, Koga-shi Ibaraki 306-0213 (JP); KOGAI, Yasumichi, Tokyo 160-0022 (JP); HATTORI, Hisashi, Tokyo 160-0022 (JP); KAWABE, Karl Kazushige, Tokyo 160-0022 (JP)
(74) Representative: Hoffmann Eitle
(86) International application number: PCT/JP2013/082867
(87) International publication number: WO 2014/103667

(57) **Abstract**

Provided is a functional nonwoven fabric that has good feeling upon use, such as hand feeling (softness) or texture, and is capable of stably exhibiting the characteristics of hydroxyapatite (for example, microbe adsorption property).

Disclosed is a functional nonwoven fabric in which sintered hydroxyapatite particles are fixed by thermal fusion bonding to a surface of a nonwoven fabric containing fibers, at least surfaces of the fibers being mainly composed of a thermoplastic resin, wherein a shearing rigidity of the functional nonwoven fabric is 8.5 gf/cm/degree or less.

## Description

### Technical Field

The present invention relates to a functional nonwoven fabric and a manufacturing method therefor, and more specifically, to a functional nonwoven fabric in which predetermined functional particles are fixed to a fiber surface and a manufacturing method therefor.

### Background Art

In recent years, as hygiene consciousness has been growing, a material having an antimicrobial property and a sterilization property is demanded. For example, a functional nonwoven fabric has been widely used in which functional particles having an antimicrobial function and a sterilization function are fixed to a nonwoven fabric that is widely used for application as a material, thereby imparting an antimicrobial property, a sterilization property, or the like to the nonwoven fabric. Here, as the functional particles having a sterilization action, hydroxyapatite particles are exemplified. Since hydroxyapatite has an excellent protein adsorption property, ion-exchange capacity, and the like, hydroxyapatite is excellent in an effect of adsorbing microbes. Microbes are adsorbed onto particles, and as a result, hydroxyapatite also has a sterilization effect. Moreover, since hydroxyapatite also has high biological affinity, hydroxyapatite is a material which is also used safely with respect to human bodies, for example, is also used as a biomaterial.

As the functional nonwoven fabric, for example, Patent Literature 1 discloses a spray for spraying a component containing a calcium phosphate compound (for example, hydroxyapatite) to a mask. According to the relevant invention, it is possible to form an antimicrobial nonwoven fabric easily. In addition, Patent Literature 2 discloses a method of chemically fixing hydroxyapatite to a polymer material by using a silane coupling agent or the like. Patent Literature 3 discloses a functional fiber aggregate in which functional particles such as hydroxyapatite are impregnated in or coated to a fiber aggregate and then the fiber aggregate (a nonwoven fabric) is subjected to a heat treatment at a temperature equal to or higher than a softening point of the fiber such that the functional particles are thermally bonded. Furthermore, Patent Literature 4 discloses a filler-fixed fiber structure obtained in such a manner that a filler dispersion obtained by dispersing a filler (solid particles or the like, for example, hydroxyapatite particles) in a solution is imparted to a fiber structure (for example, a fiber bundle) containing a heat-and-humidity gelling resin (a resin that becomes a gel by being heated in presence of moisture) to bring the heat-and-humidity gelling resin into contact with the filler, and then a steam treatment is performed on the heat-and-humidity gelling resin at a predetermined temperature so as to turn a portion of the heat-and-humidity gelling resin into a gel, thereby fixing the filler to the surface of the resin.

### Citation List

### Patent Literature

Patent Literature 1: JP H11-199403 A
Patent Literature 2: JP 3836444 B2
Patent Literature 3: JP H6-192961 A
Patent Literature 4: JP 4603898 B2

However, a functional nonwoven fabric obtained by the invention described in Patent Literature 1 has poor continuousness of a sterilization effect, for example, from the reason that hydroxyapatite is likely to be left out from between fibers. Since the method described in Patent Literature 2 is a fixing method performed by chemical bonding, there is a case where a particle having a large diameter by which large surface characteristics may be obtained increases in a volume or mass of the particle with respect to a range of a bonding face, and thus the particle is difficult to be fixed to a polymer material. Therefore, a nonwoven fabric obtained by the relevant method may not have a sufficient function in some cases. Furthermore, the functional nonwoven fabrics described in Patent Literature 3 and Patent Literature 4 have a problem that they are not suitable for direct use with respect to human bodies (for example, skin or a mucous membrane) in terms of feeling upon use, such as hand feeling (softness) or texture.

### Summary of Invention

### Technical Problem

In this regard, the invention is intended to provide a functional nonwoven fabric that has good feeling upon use, such as hand feeling (softness) or texture, and is capable of stably exhibiting the characteristics (for example, a microbe adsorption property) of hydroxyapatite, and a manufacturing method therefor.

### Solution to Problem

The present inventors conducted intensive studies in order to solve the above-described problems. As a result, they found that a functional nonwoven fabric having a predetermined shearing rigidity to which sintered hydroxyapatite particles are fixed can be obtained by fixing the sintered hydroxyapatite particles to a nonwoven fabric using a specific method and thus the surface characteristics (for example, a microbe adsorption property) of the hydroxyapatite particles are easily exerted and the particles are stably held in the fibers. For these reasons, the functional nonwoven fabric is a functional nonwoven fabric which can exhibit a high sterilization function for a long period of time, has good feeling upon use, such as hand feeling or texture, and is also suitable for use with respect to human bodies. Accordingly, they completed the invention.

That is, according to the invention, there is provided a functional nonwoven fabric in which sintered hydroxyapatite particles are fixed by thermal fusion bonding to a surface of a nonwoven fabric containing fibers, at least surfaces of the fibers being mainly composed of a thermoplastic resin, wherein a shearing rigidity of the functional nonwoven fabric is 8.5 gf/cm/degree or less.

In the functional nonwoven fabric, it is preferable that an aspect ratio of the sintered hydroxyapatite particle is 1.45 or less.

In the functional nonwoven fabric, it is preferable that a particle diameter of the sintered hydroxyapatite particle is 0.1 µm or more but 10 µm or less.

The functional nonwoven fabric may be intended for oral use.

According to the invention, there is provided a method for manufacturing a functional nonwoven fabric, the method including blowing an airflow containing sintered hydroxyapatite particles, which have been heated to a temperature higher than a melting point of a thermoplastic resin, to a surface of a nonwoven fabric containing fibers, at least surfaces of the fibers being mainly composed of the thermoplastic resin, so that the sintered hydroxyapatite particles are fixed by thermal fusion bonding to the surface of the nonwoven fabric.

In the method for manufacturing a functional nonwoven fabric, it is preferable that an aspect ratio of the sintered hydroxyapatite particle is 1.45 or less.

In the method for manufacturing a functional nonwoven fabric, it is preferable that a particle diameter of the sintered hydroxyapatite particle before being blown to the surface of the nonwoven fabric is 0.1 µm or more but 10 µm or less.

In the method for manufacturing a functional nonwoven fabric, the functional nonwoven fabric may be intended for oral use.

### Advantageous Effects of Invention

According to the invention, it is possible to provide a functional nonwoven fabric that has good feeling upon use, such as hand feeling (softness) or texture, and is capable of stably exhibiting the characteristics (for example, a microbe adsorption property) of hydroxyapatite, and a manufacturing method therefor.

### Brief Description of Drawings

Fig. 1 is a photograph illustrating an exemplary embodiment before using a functional nonwoven fabric according to an embodiment of the invention.
Fig. 2 is a photograph illustrating an exemplary embodiment in the case of using the functional nonwoven fabric according to the embodiment of the invention.
Fig. 3 is a graph illustrating an XRD spectrum of sintered hydroxyapatite particles used in Examples 1 to 6 and Comparative Examples 2 to 3.
Fig. 4 is a graph illustrating an XRD spectrum of unsintered hydroxyapatite particles used in Comparative Example 1.

### Mode for Carrying Out the Invention

Hereinafter, a structure, a manufacturing method, physical properties, and an application of a functional nonwoven fabric according to an embodiment of the invention will be sequentially described, but the embodiment of the invention is not limited thereto.

### <<Structure of Functional Nonwoven Fabric>>

First, the structure of a functional nonwoven fabric according to an embodiment of the invention will be described. The functional nonwoven fabric according to the embodiment of the invention is a nonwoven fabric having a predetermined function which is obtained by fixing hydroxyapatite particles to the surface of the nonwoven fabric by thermal fusion bonding. Here, the expression "particles are fixed to the surface of the nonwoven fabric by thermal fusion bonding" means that a resin on at least surfaces of fibers constituting the nonwoven fabric contacting with solid particles (hereinafter, referred to as "constituent fibers of the nonwoven fabric") is melted by heat and then solidified again such that the particles are fixed to the constituent fiber surface of the nonwoven fabric, and as a result, the particles become a state in which the particles are fixed to the surface of the nonwoven fabric. In this case, most of the particles are fixed in a form in which part of the particles are buried in the fibers. The expression "the nonwoven fabric having a predetermined function" in the embodiment of the invention means that a nonwoven fabric to which a function of hydroxyapatite particles to be described later is imparted. Hereinafter, hydroxyapatite particles that are constituent materials of the functional nonwoven fabric according to the embodiment of the invention and a nonwoven fabric that is a base material (hereinafter, referred to as the "base nonwoven fabric") will be described in detail.

### <Hydroxyapatite Particle>

First, hydroxyapatite (calcium phosphate hydroxide) particles according to the embodiment of the invention will be described. Incidentally, physical properties (an aspect ratio and a particle diameter) of the hydroxyapatite particles described herein indicate physical properties of hydroxyapatite particles in a state in which the particles are fixed to the surface of the nonwoven fabric (since there is a case where a shape change or the like may occur depending on a manufacturing stage, it cannot be said that the physical properties of hydroxyapatite particles in a state in which the particles are fixed to the surface of the nonwoven fabric and the physical properties of hydroxyapatite particles as constituent materials of the functional nonwoven fabric are always the same).

### (Composition)

Hydroxyapatite (HAp) is basic calcium phosphate represented by the chemical formula: Ca₁₀(PO₄)₆(OH)₂, and is naturally present as a main component of a tooth or a bone, or as mineral.

### (Function and Characteristics)

The hydroxyapatite particle exhibits a high biological affinity. In particular, the hydroxyapatite particle has a high affinity to bones and cells are likely to infiltrate into a porous sintered body. Thus, when the hydroxyapatite particle is applied to a defect portion of a bone, autologous bones are promoted to form. In addition, the hydroxyapatite particle exhibits a high adsorption property with respect to amino acids, protein (for example, microbes), lipid, sugar, and the like. The hydroxyapatite has excellent ion-exchange capacity, and a calcium ion group is exchanged to a cation and a phosphate group or a hydroxide ion group is exchanged to an anion respectively. For example, when a medical agent containing fluoride ions is applied to teeth, ion exchange occurs between the fluoride ions and hydroxide ions of the hydroxyapatite, thereby imparting acid resistivity to surfaces of the teeth.

### (Obtaining Method)

As a general method for manufacturing hydroxyapatite particles, for example, a solution method (a wet method) is exemplified. This method is a method of performing synthesis by allowing calcium ions and phosphate ions to react to each other in a neutral or alkaline aqueous solution, and examples thereof include a method of utilizing a neutralization reaction or a method of allowing a calcium salt and a phosphate salt to react to each other. Incidentally, it is also possible to obtain larger diameter (aggregated) or denser particles by sintering primary particles, for example. In addition, for example, various hydroxyapatite particles such as micro-SHAp (IHM-100P000, SofSera Corporation) or hydroxyapatite (produced by Taihei Chemical Industrial Co., Ltd.) are commercially available and various manufacturing methods therefor, and shapes, characteristics, or the like thereof are also available.

### (Sintering)

As the hydroxyapatite particles fixed to the surface of the base nonwoven fabric, sintered hydroxyapatite particles (hereinafter, unless otherwise specified, "hydroxyapatite particles" indicate "sintered hydroxyapatite particles") are used. When the hydroxyapatite particles are sintered (for example, at 800°C for 1 hour), the crystallinity of particles increases and an aggregate of a plurality of primary particles is fused by heat, thereby obtaining more robust and stable particles. In a case where particles fixed to the base nonwoven fabric are unsintered hydroxyapatite, the particles are likely to be collapsed in a using method in which a strong load is applied to the functional nonwoven fabric (for example, a wiping-off application). For this reason, the collapsed particles act like polishing components and thus texture may be deteriorated over time. On the other hand, in a case where particles fixed to the base nonwoven fabric are sintered hydroxyapatite, the particles are less likely to be collapsed even in the relevant using method and texture is further improved. Moreover, it is considered that the characteristics (for example, a microbe adsorption property) of the functional nonwoven fabric may be maintained stably for a longer period of time. Incidentally, whether or not the hydroxyapatite particles are sintered can be determined by a crystalline degree of the particles. The crystalline degree of the hydroxyapatite particles can be measured by an X-ray diffraction (XRD) method, and as a half-value width of a peak representing each crystalline plane is narrower, it can be said that the crystallinity is high. Specifically, the sintered hydroxyapatite particles in this embodiment indicate high crystallinity hydroxyapatite particles having a half-value width of 0.8 or less (preferably, 0.5 or less) at d = 2.814.

### (Aspect Ratio)

An aspect ratio of the hydroxyapatite particle according to the embodiment of the invention is preferably 1.45 or less. When the aspect ratio of the hydroxyapatite particle is 1.45 or less, the shapes of the hydroxyapatite particles fixed to the surface of the nonwoven fabric become regular to some extent and the contact manners of the particles to a target surface become uniform to some extent. Therefore, it is possible to obtain a functional nonwoven fabric with excellent texture. In particular, when the shape of the hydroxyapatite particle is a massive shape (preferably, a substantially spherical shape), the functional nonwoven fabric with excellent texture is obtained. In order to further improve the relevant effect, the aspect ratio of the hydroxyapatite particle is preferably 1.35 or less, more preferably 1.25 or less, and particularly preferably 1.2 or less. Here, a measuring method of the aspect ratio of the hydroxyapatite particle fixed to the surface of the nonwoven fabric is performed according to the following measuring method (1).

### (Measuring Method 1)

In SEM images obtained by imaging the hydroxyapatite particles fixed to the constituent fibers of the nonwoven fabric, a particle imaged from substantially above, which is not protruded from both ends of the fiber in a radial direction, is selected. Subsequently, two line segments in which both ends thereof are located on the outer circumference of the particle are drawn on the particle. At this time, one line segment has the maximum length. The other line segment is drawn at the center of the one line segment such that the one line segment is intersected with the other line segment. Of the two line segments drawn in this way, a length of the line segment having a short length is designated as a minor axis, a length of the line segment having a long length is designated as a major axis, and a ratio of major axis/minor axis is obtained. Furthermore, an average value of the ratio of major axis/minor axis in 150 particles, which are selected in order from the longest major axis to the shortest major axis, is obtained to be set as an aspect ratio. However, a particle which is shown that the outline thereof is blurred, a particle which is too close to another particle so that the boundary thereof is vague, a particle of which a part is under cover of the shadow of another particle, or the like is excluded from the measurement target. As the aspect ratio becomes closer to 1, the captured image of the particle is close to a circle or a square, and the three-dimensional shape of the particle is considered to be close to a massive shape (for example, a spherical shape).

### (Particle Diameter)

The particle diameter of the hydroxyapatite particle according to the embodiment of the invention is preferably 0.1 µm or more but 10 µm or less, more preferably 0.5 µm or more but 8 µm or less, and still more preferably 1 µm or more but 5 µm or less, in order to fix a sufficient amount of the particles to the base nonwoven fabric. The particle diameter of the hydroxyapatite particle preferably has a size of 1/3 or less with respect to a fiber diameter of the constituent fiber (this will be described later) of the base nonwoven fabric. When the particle diameter of the hydroxyapatite particle exceeds 10 µm (or exceeds a size of 1/3 with respect to a fiber diameter of the constituent fiber of the base nonwoven fabric), the solid particles are likely to be left out from the fiber surface. When the particle diameter of the hydroxyapatite particle is less than 0.1 µm, the surface area of the hydroxyapatite particle, which may come in contact with a target surface is decreased when the target surface is wiped off using the functional nonwoven fabric, and thus the functionality (for example, a microbe adsorption property) of the functional nonwoven fabric is deteriorated. Here, a method of measuring the particle diameter of the hydroxyapatite particle fixed to the nonwoven fabric is performed according to the following measuring method (2).

### (Measuring Method 2)

In a similar way to the measuring method (1), in SEM images obtained by imaging the hydroxyapatite particles fixed to the constituent fibers of the nonwoven fabric, a particle imaged from substantially above, which is not protruded from both ends of the fiber in a radial direction of the fiber, is selected. Subsequently, two line segments in which both ends thereof are located on the outer circumference of the particle are drawn on the particle.

At this time, one line segment has the maximum length. The other line segment is drawn at the center of the one line segment such that the one line segment is intersected with the other line segment. Of the two line segments drawn in this way, a length of the line segment having a short length is designated as a minor axis, a length of the line segment having a long length is designated as a major axis. Furthermore, an average value of the major axes in 150 particles, which are selected in order from the longest major axis to the shortest major axis, is obtained to be set as a particle diameter of the hydroxyapatite particle. However, a particle which is shown that the outline thereof is blurred, a particle which is too close to another particle so that the boundary thereof is vague, a particle of which a part is under cover of the shadow of another particle, or the like is excluded from the measurement target. Incidentally, the particle diameter of the hydroxyapatite particle described herein does not mean a particle diameter of a primary particle but means a particle diameter of a particle in which a plurality of primary particles are cohered.

### <Base Nonwoven Fabric>

Next, the base nonwoven fabric of the functional nonwoven fabric according to the embodiment of the invention will be described. The base nonwoven fabric according to the embodiment of the invention is not particularly limited as long as it has fibers at least surfaces of which are mainly composed of a thermoplastic resin. The base nonwoven fabric may contain only the above-described fibers or may contain other fibers. Other fibers than the above-described fibers (that is, fibers, at least surfaces of which are mainly composed of a thermoplastic resin) are not particularly limited, and it is possible to use fibers of which surfaces are not a thermoplastic resin, for example, inorganic fibers or fibers having a decomposition temperature without having a melting point. Incidentally, examples of the fibers at least surfaces of which are mainly composed of a thermoplastic resin include fibers (substantially) at least surfaces of which are composed of one or more kinds of thermoplastic resins. Here, the expression "mainly obtained from" means that the thermoplastic resin as a target is contained in 50% by mass or more with respect to the constituent resin on the fiber surface (preferably 60% by mass or more, more preferably 70% by mass or more, and particularly preferably 90% by mass or more).

### (Fiber)

The constituent fibers of the base nonwoven fabric are fibers at least surfaces of which are composed of one or two or more thermoplastic resins, and it is possible to use, as the constituent fibers of the base nonwoven fabric, single fibers having a single composition or composite fibers or the like which are obtained from a plurality of thermoplastic resins, such as core-sheath fibers in which a plurality of components are arranged in a concentric circular shape as seen in cross-section, sea-island fibers, or eccentric core-sheath fibers. Incidentally, in any cases of using the single fiber and the composite fiber, a fiber of which a fiber cross-section is a modified cross-section may be also used. Regarding the constituent fibers of the base nonwoven fabric, it is necessary that a melting point (or a softening point) of at least one resin component which is exposed from the fiber surface be equal to or lower than a melting point or a decomposition temperature of the hydroxyapatite particle to be fixed. The hydroxyapatite particle is fixed to a contact portion by contacting with the fiber surface in a state in which the temperature of the hydroxyapatite particle becomes a temperature equal to or higher than a melting point of the thermoplastic resin composing the constituent fiber surface of the base nonwoven fabric. Furthermore, as the constituent fibers of the base nonwoven fabric, fibers having a melting point equal to or higher than a melting point or a decomposition temperature of the hydroxyapatite particle, that is, fibers which do not involve the fixation of the particles may be included. Proportions of fibers which involve the fixation of the hydroxyapatite particles and fibers which do not involve the fixation thereof can be appropriately selected depending on design, and the proportion of the fibers which involve the fixation of the particles is preferably 50% by mass or more such that the functional nonwoven fabric has high functionality, that is, functions of hydroxyapatite, such as a microbe adsorption property, can be exerted in a higher level.

As such a thermoplastic resin having a melting point equal to or lower than a melting point or a decomposition temperature of the hydroxyapatite particle, specifically, polyester, polyolefin (for example, polyethylene or polypropylene), polyamide, and the like are preferably used. Copolymerized polyester, copolymerized polypropylene, copolymerized polyethylene (for example, an ethylene-ethylene-vinyl acetate copolymer), and the like can be preferably used. These resins can be appropriately selected depending on the use application. For example, in a case where using for skin, a mucous membrane, or the like is assumed, resins having biological compatibility such as polyglycolic acid, polylactic acid, a polylactide/polyglycolide copolymer, and polydioxane can be used. Examples of a composite fiber obtained by the composite of two or more types of resins each having a different melting point may include composite fibers obtained by the combination of resins such as copolymerized polyester/polyester, copolymerized polypropylene/polypropylene, polypropylene/polyamide, polyethylene/polypropylene, polypropylene/polyester, or polyethylene/polyester, copolymerized polyethylene /polyethylene, and copolymerized polyethylene/polypropylene. In a case where the composite fiber is a core-sheath type composite fiber having a high melting point resin at its core and a low melting point resin at its sheath, the shrinkage or broken thread of the fiber is further less likely to occur when the solid particles are fixed to the fiber surface, which is favorable. As such a core-sheath type composite fiber, a composite fiber composed of a high melting point polyester (for example, a melting point of about 255°C) and a low melting point polyester (for example, a melting point of about 110°C), a composite fiber composed of polypropylene (a melting point of 160°C) and high density polyethylene (a melting point of 130°C), or the like is exemplified.

The fiber may be a fiber in which a thermoplastic resin is applied as the sheath portion, for example, by coating or the like, to the surface of the fiber in which the core portion does not have a melting point but has a decomposition temperature (for example, a fiber such as a rayon fiber, an acetate fiber, a wool fiber, or a carbon fiber). Furthermore, the fiber may be a fiber in which a thermoplastic resin is applied as the sheath portion, for example, by coating or the like, to the surface of the fiber in which the core portion is an inorganic fiber and has a high melting point (for example, a fiber such as a glass fiber, a ceramic fiber, or a metal fiber).

The thermoplastic resin on the surface may be modified by a well-known technique, and for example, can be hydrophilized by a plasma treatment or can be bonded with an arbitrary surface functional group by graft polymerization.

The average diameter of the constituent fiber of the base nonwoven fabric is preferably in a range of 1 µm to 100 µm, and more preferably in a range of 2 µm to 50 µm. When the average diameter of the constituent fiber of the base nonwoven fabric is less than 1 µm, the strength of the base nonwoven fabric tends to be decreased. In particular, in the case of using this base nonwoven fabric for oral use (particularly, for bacterial eradication in tooth), the strength thereof is likely to become insufficient. In a case where the hydroxyapatite particle having a large particle diameter is fixed, the particle tends to be difficult to be fixed. On the other hand, when the average diameter exceeds 100 µm, rough feeling occurs in the base nonwoven fabric, which is not favorable. Here, the average diameter of the fiber is obtained in such a manner that, using a scanning electron microscope or the like, an image of the main surface of the base nonwoven fabric is enlarged and imaged, regarding a fiber diameter (a length in a direction perpendicular to the longitudinal direction of the fiber) which may be confirmed in the image of the fiber, arbitrary 500 or more places in the fibers are sampled to obtain a number average fiber diameter, and then the number average fiber diameter is designated as the average diameter of the fiber. The cross-sectional shape or the surface state of the fiber can be set to an arbitrary shape or state. For example, the fiber can be a fiber in which the cross-sectional shape of the composite fiber obtained from the thermoplastic resin is divided in wedge shape by a mechanical stress of water jet or the like. Moreover, the fiber can also be a porous fiber having an unevenness on the fiber surface. Furthermore, the fiber can also be a fiber in which the composite fiber obtained from the thermoplastic resin is divided in a fibrillar shape by a mechanical stress of water jet or the like.

### (Shape)

The shape of the base nonwoven fabric according to the embodiment of the invention is not particularly limited, and examples thereof may include an elongated fiber sheet (for example, a fiber sheet wound on a roll) or a non-elongated fiber sheet (that is, a fiber sheet which can be obtained by cutting the elongated fiber sheet). An area density of the base nonwoven fabric is preferably 5 g/m² or more but 150 g/m² or less, more preferably 10 g/m² or more but 100 g/m² or less, and still more preferably 20 g/m² or more but 90 g/m² or less. When the area density of the base nonwoven fabric exceeds 150 g/m², in the case of using it particularly for oral use, feeling upon use (for example, texture) may be deteriorated. When the area density of the base nonwoven fabric is less than 5 g/m², the strength of the base nonwoven fabric to be obtained is weak and thus the base nonwoven fabric is inferior in practicality. In addition, a sufficient amount of the hydroxyapatite particle cannot be fixed to the base nonwoven fabric and thus the function (a microbe adsorption property or the like) of the functional nonwoven fabric to be obtained may be deteriorated in some cases.

### <<Physical Property of Functional Nonwoven Fabric>>

Next, the physical properties of the functional nonwoven fabric according to the embodiment of the invention will be described. The functional nonwoven fabric according to the embodiment of the invention has a predetermined shearing rigidity. Since the functional nonwoven fabric has this physical property, it is possible to make feeling upon use, such as hand feeling (softness) or texture, favorable.

### <Shearing Rigidity>

The shearing rigidity of the functional nonwoven fabric according to the embodiment of the invention, which is measured according to the following measuring method (3) is 8.5 gf/cm/degree or less. When the shearing rigidity of the functional nonwoven fabric is within the above range, it is possible to obtain excellent feeling upon use, such as hand feeling (softness), and even in a case where the functional nonwoven fabric is applied to a three-dimensional surface (a wiping-off application or the like), excellent followability is achieved. In order to exert these effects more effectively, the shearing rigidity is preferably 8.0 gf/cm/degree or less and more preferably 7.5 gf/cm/degree or less. Incidentally, a lower shearing rigidity of the functional nonwoven fabric according to the embodiment of the invention is more preferable. A lower limit value thereof is not particularly limited, and for example, may be set to 1.0 gf/cm/degree or more.

### (Measuring Method 3)

The functional nonwoven fabric as a measurement target is cut into a size of 20 x 20 (cm), is set on a tensile shear tester having an inter-chuck distance of 5 cm (for example, manufactured by KATO TECH CO., LTD., KES-FB1), and a tension of 10 g/cm is applied thereto. Next, shearing is performed up to a shear angle 8° and then shearing is performed up to a shear angle 8° in the opposite direction. A shearing rigidity is obtained by using a change in shear force per unit width with respect to a change in this shear angle. This shearing rigidity is measured three times for each of the longitudinal direction and the cross direction of the functional nonwoven fabric, and an average value thereof is designated as a shearing rigidity G.

### <Coefficient of Static Friction>

In the functional nonwoven fabric of the invention, a coefficient of static friction of the functional nonwoven fabric, which is measured according to the following measuring method (4), is preferably 0.25 or less, more preferably 0.24 or less, and still more preferably 0.22 or less. In a case where the functional nonwoven fabric having such a coefficient of static friction is used with respect to a delicate portion such as skin, a mucous membrane, or the like of humans, for example, used for a wiping-off application, a target to be wiped off is less likely to be damaged and feeling upon use, such as texture or hand feeling (softness), is excellent. Incidentally, a lower limit value of the coefficient of static friction is not particularly limited, and for example, may be 0.10 or more.

### (Measuring Method 4)

An average value based on the results of 20 times measurement is obtained by performing a test using a friction meter, such as Portable Friction Meter Muse TYPE 94i manufactured by Shinto Scientific Co., Ltd., as a measuring instrument, and then the average value is designated as the coefficient of static friction.

### <<Manufacturing Method for Functional Nonwoven Fabric>>

Hereinbefore, the structure and the physical properties of the functional nonwoven fabric according to the embodiment of the invention have been described in detail. Subsequently, a method for manufacturing a functional nonwoven fabric having the structure and the physical properties described above will be described. The method for manufacturing a functional nonwoven fabric according to the embodiment of the invention includes, mainly, a step of fixing the hydroxyapatite particles to the surface of the base nonwoven fabric by thermal fusion bonding (hereinafter, referred to as the "particle fixing step"). Hereinafter, as the method for manufacturing a functional nonwoven fabric according to the embodiment of the invention, the hydroxyapatite particles and the base nonwoven fabric that are raw materials of the functional nonwoven fabric and the particle fixing step will be described in detail. In addition, a pre-treatment step that is an additional step will be described.

### <Hydroxyapatite Particle>

### (Composition, Function · Characteristics and Obtaining Method of Hydroxyapatite Particle)

The composition, the function · characteristics, the obtaining method, and the like of hydroxyapatite particles in the method for manufacturing a functional nonwoven fabric according to the embodiment of the invention are as having been already described above. Therefore, here, the detailed description thereof is not provided.

### (Sintering of Hydroxyapatite Particle)

In the method for manufacturing a functional nonwoven fabric according to the embodiment of the invention, sintered hydroxyapatite particles are used. When the hydroxyapatite particles are sintered (for example, at 800°C for 1 hour), the crystallinity of particles increases and an aggregate of a plurality of primary particles is fused by heat, thereby obtaining more hard and stable particles. When such hard and stable sintered hydroxyapatite particles are fixed to the base nonwoven fabric, the particles are less likely to be collapsed even in a using method in which a strong load is applied to the functional nonwoven fabric (for example, a wiping-off application) and texture is further improved. Moreover, the characteristics (for example, a microbe adsorption property) of the functional nonwoven fabric can be maintained stably for a longer period of time. On the other hand, in a case where particles fixed to the base nonwoven fabric are unsintered hydroxyapatite, since the particles are likely to be collapsed in a using method in which a strong load is applied to the functional nonwoven fabric, the collapsed particles act like polishing components and thus texture may be deteriorated over time. The surfaces of the unsintered hydroxyapatite particles have slightly sticky, and thus there is a concern that, when the airflow containing the relevant particles are blown to the base nonwoven fabric, the particles are attached to each apparatus place or a pipe which is disposed on the process path, and thus this causes a trouble, thereby decreasing productivity. Incidentally, the determination whether or not the hydroxyapatite particles are sintered as having been already described above.

### (Aspect Ratio and Shape of Hydroxyapatite Particle)

Here, as the hydroxyapatite particle in the method for manufacturing a functional nonwoven fabric according to the embodiment of the invention, the hydroxyapatite particle having an aspect ratio of 1.45 or less is preferably used. In a case where the aspect ratio of the hydroxyapatite particle is 1.45 or less, even when the airflow containing heated hydroxyapatite particles is brought into contact with the base nonwoven fabric, since the particle has an isotropic shape, the contact surface of the particle with the constituent fiber of the base nonwoven fabric is easily and stably secured, and thus the particle is easily and stably fixed to the base nonwoven fabric. For this reason, the quality of the manufactured functional nonwoven fabric is improved (for example, a microbe adsorption property or the like is maintained for a long period of time or the fixing strength of the particle with respect to a load such as wiping-off is improved). In order to further improve the relevant effect, the aspect ratio of the hydroxyapatite particle is preferably 1.35 or less, more preferably 1.25 or less, and particularly preferably 1.2 or less. As the aspect ratio becomes closer to 1, the captured image of the particle is close to a circle or a square, and the three-dimensional shape of the particle is considered to be close to a massive shape (for example, a spherical shape). In particular, when the particle is substantially spherical, the above-described effect is more improved.

The aspect ratio of the hydroxyapatite particle described herein is a numerical value obtained by the following method. In SEM images obtained by imaging the hydroxyapatite particles, two line segments in which both ends thereof are located on the outer circumference of the particle are drawn on the particle. At this time, one line segment has the maximum length. The other line segment is drawn at the center of the one line segment such that the one line segment is intersected with the other line segment. Of the two line segments drawn in this way, a length of the line segment having a short length is designated as a minor axis, a length of the line segment having a long length is designated as a major axis, and a ratio of major axis/minor axis is obtained. Furthermore, an average value of the ratio of major axis/minor axis in 150 particles, which are selected in order from the longest major axis to the shortest major axis, is obtained to be set as an aspect ratio. However, a particle which is shown that the outline thereof is blurred, a particle which is too close to another particle so that the boundary thereof is vague, a particle of which a part is under cover of the shadow of another particle, or the like is excluded from the measurement target.

### (Particle Diameter of Hydroxyapatite Particle)

In the method for manufacturing a functional nonwoven fabric according to the embodiment of the invention, the particle diameter of the hydroxyapatite particle before being blown to the surface of the base nonwoven fabric is preferably 0.1 µm or more but 10 µm or less, more preferably 0.5 µm or more but 8 µm or less, and still more preferably 1 µm or more but 5 µm or less. Furthermore, the particle diameter of the hydroxyapatite particle before being blown to the surface of the base nonwoven fabric preferably has a size of 1/3 or less with respect to a fiber diameter of the constituent fiber of the base nonwoven fabric according to the embodiment of the invention. When the particle diameter of the hydroxyapatite particle is within such a range, the particles are less likely to be left out when the particles are fixed to the constituent fibers of the base nonwoven fabric, and the particles are fixed properly. Therefore, it is possible to obtain a functional nonwoven fabric having an excellent function (for example, a microbe adsorption property). Incidentally, the particle diameter of the hydroxyapatite particle described herein is a numerical value obtained by the following method. In SEM images obtained by imaging the hydroxyapatite particles, two line segments in which both ends thereof are located on the outer circumference of the particle are drawn on the particle. At this time, one line segment has the maximum length. The other line segment is drawn at the center of the one line segment such that the one line segment is intersected with the other line segment. Of the two line segments drawn in this way, a length of the line segment having a short length is designated as a minor axis, a length of the line segment having a long length is designated as a major axis. Furthermore, an average value of the major axes in 150 particles, which are selected in order from the longest major axis to the shortest major axis, is obtained to be set as a particle diameter.

### <Base Nonwoven Fabric or the Like>

### (Fiber and Shape of Base Nonwoven Fabric)

In the method for manufacturing a functional nonwoven fabric according to the embodiment of the invention, the fiber, the shape, and the like of the base nonwoven fabric are as having been already described above. Therefore, here, the detailed description thereof is not provided.

### (Method for Manufacturing Base Nonwoven Fabric)

As the method for manufacturing a base nonwoven fabric, a conventional method for manufacturing a nonwoven fabric such as a dry method, a spun-bonded method, a melt blown method, a flash spinning method, a carding method using a short fiber, a wet papermaking method, or the like is exemplified, and a nonwoven fabric produced by these methods can be used as a base nonwoven fabric. A base nonwoven fabric in which the fibers are bonded can be obtained in such a manner that a composite fiber or the like obtained by the composite of two or more types of resins each having a different melting point is mixed in advance with a fiber web formed by these manufacturing method, and then a heating treatment is performed. Furthermore, it is also possible to obtain a base nonwoven fabric by entangling the fiber web by a mechanical entangling treatment. Incidentally, the mechanical entangling treatment of the base nonwoven fabric is preferably an entangling treatment by hydroentangling, needle punching, or the like. When such an entangling treatment is used for the base nonwoven fabric, the hand feeling (softness) of the base nonwoven fabric can be improved. In addition, it is also possible to obtain a base nonwoven fabric by passing the fiber web through a smooth roll and a roll with an unevenness so that the fiber web is partially bonded. Fiber webs or nonwoven fabrics of which types are different may be laminated or integrally formed (unified).

### <Particle Fixing Step>

In the particle fixing step, by blowing an airflow containing heated hydroxyapatite particles, which have been heated to a temperature higher than a melting point of a thermoplastic resin, to a surface of a nonwoven fabric (base nonwoven fabric) containing fibers at least surfaces of which are mainly composed of the thermoplastic resin, the hydroxyapatite particles are fixed by thermal fusion bonding to the surface of the base nonwoven fabric. More specifically, the hydroxyapatite particles are heated to a temperature higher than a melting point of the thermoplastic resin, the hydroxyapatite particles are brought into contact with the constituent fiber of the base nonwoven fabric in a state in which the temperature of the hydroxyapatite particles is maintained to be higher than a melting point of the thermoplastic resin, and the resin on the fiber surface is melted by heat. Thereafter, the resin is cooled and solidified again, thereby fixing the hydroxyapatite particles to the constituent fiber surface of the base nonwoven fabric (that is, the surface of the base nonwoven fabric).

In this way, in order to prepare an airflow mixed with the heated hydroxyapatite particles, for example, (a mixed airflow preparation method a) a method of supplying hydroxyapatite particles, which are heated to a temperature equal to or higher than a melting point of the thermoplastic resin, into an airflow; (a mixed airflow preparation method b) a method of supplying hydroxyapatite particles into an airflow which is heated to a temperature equal to or higher than a melting point of the thermoplastic resin; or (a mixed airflow preparation method c) a method of supplying hydroxyapatite particles into an airflow, followed by being heated to a temperature equal to or higher than a melting point of the thermoplastic resin can be exemplified.

Among these, according to the mixed airflow preparation method (a), since there is no case where the airflow heated to a temperature equal to or higher than a melting point of the thermoplastic resin (the resin on at least the surface of the constituent fiber of the base nonwoven fabric) is brought into contact with the base nonwoven fabric itself, the shrinkage or the like of the fiber is less likely to occur, which is favorable. Therefore, even before blowing the airflow, it is preferable to adjust the temperature thereof to be a temperature lower than a melting point of a thermoplastic resin having the lowest melting point.

Incidentally, in the method for manufacturing a functional nonwoven fabric according to the embodiment of the invention, it is necessary to heat the hydroxyapatite particle to a temperature equal to or higher than a melting point of the thermoplastic resin. However, in a case where a problem arises in which broken thread or shrinkage of the fiber is caused by the fixation of the hydroxyapatite particle having an excessively high temperature to the fiber, it is preferable to heat the hydroxyapatite particle to a temperature not exceeding a temperature higher than a melting point of the thermoplastic resin by 100°C, and more preferable to heat the hydroxyapatite particle to a temperature not exceeding a temperature higher than a melting point of the thermoplastic resin by 50°C.

In the mixed airflow preparation method (a), preferable is a method of supplying the hydroxyapatite particles heated to a temperature equal to or higher than a melting point of the thermoplastic resin to the airflow heated to a temperature equal to or higher than the temperature, which is lower than a melting point of the thermoplastic resin by 50°C. In this case, when the airflow and the hydroxyapatite particles are mixed, there is an effect of preheating the hydroxyapatite particles such that the temperature of the hydroxyapatite particle is not decreased to a temperature lower than a melting point of the thermoplastic resin. There is an effect of warming the hydroxyapatite particles such that the temperature of the hydroxyapatite particle is not decreased to a temperature lower than a melting point of the thermoplastic resin until the heated hydroxyapatite particles come into collision with the fiber. Incidentally, if a mixed airflow of the airflow and the hydroxyapatite particles is blown to the fiber, in a case where a problem arises in which broken thread or shrinkage of the fiber is caused by the contact of the airflow having an excessively high temperature with the fiber, it is preferable to use an airflow heated to a temperature or higher, which is equal to or lower than a melting point of the thermoplastic resin by 50°C, and heated to a temperature equal to or lower than a temperature of the heated hydroxyapatite particle.

In order to obtain the heated airflow, for example, it is possible to use a method in which an airflow is generated by an airflow generating means (for example, a blower or a compressor), and then the airflow is heated to a predetermined temperature (a temperature higher than the temperature, which is lower than a melting point of the thermoplastic resin by 50°C) by a well-known heating means. In order to obtain the heated hydroxyapatite particles, for example, it is possible to use a method in which a heater is installed inside and outside a hydroxyapatite particle supplying means (for example, a hopper or a supply container), and then the hydroxyapatite particles in the hydroxyapatite particle supplying means are heated to a predetermined temperature (for example, a temperature equal to or higher than a melting point of the thermoplastic resin), or a method in which the hydroxyapatite particles are heated to a predetermined temperature (for example, a temperature equal to or higher than a melting point of the thermoplastic resin) by using a device such as a fluidized-bed type dryer which is generally used as a dryer for powder.

As a method of supplying the hydroxyapatite particles to the airflow to prepare a mixed airflow, for example, it is possible to exemplify a method of supplying the hydroxyapatite particle at a constant amount from the particle supplying means (for example, a hopper or a supply container) into the airflow, or a method in which the hydroxyapatite particles are heated up to a temperature equal to or higher than a melting point of the thermoplastic resin by using a device such as a fluidized-bed type dryer, then a mixed airflow obtained by dispersing and mixing the heated hydroxyapatite particle in the airflow by the fluidized-bed type dryer is brought out, and then the mixture is supplied to the airflow.

In addition to these methods, it is also possible to use a method in which the particle mixing means is an ejector, the airflow generated in a blower and a heating pipe serving as the airflow generating means is sent to the particle mixing means, as a particle supplying means a funnel-shaped supply container, a rotary supply controlling rotor, and a supply pipe in the particle mixing means are allowed to be connected with one another, the hydroxyapatite particles supplied from the particle supplying means are sucked by a suction power generated by the airflow, and the hydroxyapatite particles are supplied into the airflow. In this case, when the cross-sectional area of the airflow of a portion to which the hydroxyapatite particles are supplied in the particle mixing means is decreased to be smaller than the cross-sectional area therebefore and thereafter so as to increase a speed of the airflow, the suction power acts strongly, and thus an effect of dispersing and mixing the hydroxyapatite particles can be improved.

For example, it is also possible to use a method in which the particle mixing means is an ejector, the airflow generated in a blower and a heating pipe serving as the airflow generating means is sent to the particle mixing means, a mixed airflow obtained by dispersing and mixing the hydroxyapatite particle in heated airflow by the fluidized-bed type dryer serving as the particle supplying means is sent in the particle mixing means, the mixed airflow supplied from the particle supplying means is sucked by a suction power generated by the airflow, and the hydroxyapatite particles are supplied into the airflow.

Regarding a method of blowing an airflow to the base nonwoven fabric (the constituent fibers of the base nonwoven fabric), for example, when a mixed airflow containing hydroxyapatite particles is ejected from a nozzle serving as an ejecting means, the hydroxyapatite particles come into collision with the constituent fiber surface of the base nonwoven fabric by the inertia force caused due to kinetic energy imparted at the time of ejection. The ejecting means can be connected, for example, directly with the particle mixing means or connected with the particle mixing means through a connection pipe. The nozzle can be formed in a shape suitable for ejecting a fluid. For example, in order to increase the fictitious force of the hydroxyapatite particle, the nozzle can be formed in a shape having a narrow flow passage, or in order to widen an ejection angle of the hydroxyapatite particle, the nozzle can be formed in a shape in which a tip end of the nozzle is widened. It is also preferable that the nozzle be formed by selecting a material in which abrasion or the like is less likely to occur depending on the hydroxyapatite particle ejected from the nozzle.

When a method of blowing an airflow containing heated hydroxyapatite particles to the base nonwoven fabric (the constituent fibers of the base nonwoven fabric) is used as a method of contacting heated hydroxyapatite particles with the base nonwoven fabric (the constituent fibers of the base nonwoven fabric), it is preferable that the airflow containing heated hydroxyapatite particles is blown to the base nonwoven fabric supported by a movable nonwoven fabric supporting means. Such a supporting means is not particularly limited as long as a blowing treatment of the airflow containing heated hydroxyapatite particles can be performed. Preferred examples of the supporting means may include a rotation roll on which the base nonwoven fabric is placed backward and forward a treatment region for blowing the airflow containing heated hydroxyapatite particles, a tenter-type device which moves in the blowing treatment region while both sides of the base nonwoven fabric are gripped by pins or clips, a pair of rolls which hold the base nonwoven fabric there between so as to support the base nonwoven fabric backward and forward the blowing treatment region, or a supporting body with openings (for example, a conveyor net) which is capable of performing a blowing treatment in the blowing treatment region while the base nonwoven fabric is placed thereon. Incidentally, in case using a conveyor net or the like, it is also possible to support a plurality of base nonwoven fabrics at the same time.

In a case where the airflow containing heated hydroxyapatite particles is blown to the base nonwoven fabric supported by the supporting means, in order to uniformly blow the airflow in a width direction of the base nonwoven fabric, a plurality of ejecting means of an airflow containing heated hydroxyapatite particles may be installed or a plurality of nozzle holes provided in the ejecting means may be provided. Moreover, the nozzle hole may be formed in a slit shape and then in a shape in which a tip end of the nozzle extends to the whole width of the base nonwoven fabric. If the ejecting means can reciprocatively move in approximately parallel to the width direction of the base nonwoven fabric and in perpendicular or at a certain angle to the movement direction, even when the number of the ejecting means is small, the whole base nonwoven fabric can be treated.

It is preferable that, after the airflow containing heated hydroxyapatite particles is blown to the base nonwoven fabric, redundant hydroxyapatite particles which have not been fixed to the base nonwoven fabric (the constituent fiber of the base nonwoven fabric) be collected, and the collected hydroxyapatite particle be reused. As such a collecting method, it is possible to exemplify a method in which the blowing of the airflow containing heated hydroxyapatite particles to the base nonwoven fabric is performed in a fixation treatment chamber formed as an enclosed space such that redundant hydroxyapatite particles are not scattered outside the fixation treatment chamber, a particle collection box serving as a hydroxyapatite particle collecting means is connected to the fixation treatment chamber, and the redundant hydroxyapatite particles are collected by the particle collection box. In order to remove the redundant hydroxyapatite particles which have not been fixed to the base nonwoven fabric (the constituent fiber of the base nonwoven fabric), for example, it is possible to concurrently use a method in which a conveyor net is inclined and then the redundant hydroxyapatite particles are dropped by vibration, and a method of using a particle collecting means for blowing away the redundant hydroxyapatite particles by an airflow.

Incidentally, a method in which the hydroxyapatite particles are brought into contact with the constituent fibers of the base nonwoven fabric to form fibers to which the hydroxyapatite particle are thermally bonded, and then the fibers are cooled is not particularly limited as long as cooling can be performed to a temperature at which the hydroxyapatite particles can be fixed to the fiber surface, and for example, it is possible to exemplify a method of leaving the fibers at room temperature, a method of using an appropriate cooling means depending on needs, or the like.

According to the method for manufacturing a functional nonwoven fabric according to the embodiment of the invention, since the heated hydroxyapatite particles are brought into contact with the surface of the constituent fiber of the base nonwoven fabric, only a portion where the hydroxyapatite particles come in contact with the fiber surface is melted so that the hydroxyapatite particles are fixed to the fiber surface. For this reason, a case where a surface of the hydroxyapatite particle other than the contact portion or other than the fixed portion is covered with a molten resin is very less likely to occur. Moreover, a case where the hydroxyapatite particles are buried by melting and fluidizing the whole resin on the fiber surface is very less likely to occur. A problem does not arise in which the molten resin is oozed from a gap between the contacted hydroxyapatite particles, the hydroxyapatite particles located outside the hydroxyapatite particles are also fixed, the hydroxyapatite particles are partially multi-layered on the fiber surface, and thus the hydroxyapatite particles are not uniformly supported on the fiber surface. That is, basically, the hydroxyapatite particles are uniformly fixed in a single layer form.

Incidentally, the manufacturing method described herein is merely an example, and is not particularly limited as long as it is a method by which a functional nonwoven fabric, to which the hydroxyapatite particles are fixed by thermal fusion bonding, can be obtained. For example, instead of blowing the airflow containing hydroxyapatite particle, the hydroxyapatite particles heated to the temperature described above are freely dropped to the base nonwoven fabric, and thus the hydroxyapatite particles may be brought into contact with the constituent fibers of the base nonwoven fabric. In this case, for example, the base nonwoven fabric is placed on a moving conveyor and then the heated hydroxyapatite particles are, for example, sprayed from the upper portion of the conveyor. According to this, the heated hydroxyapatite particles come in contact with the constituent fiber surface of the base nonwoven fabric, and at the same time, the heated hydroxyapatite particles are maintained in a state in which the heated hydroxyapatite particles melt only the contact point of the thermoplastic resin (the constituent fiber of the base nonwoven fabric). Subsequently, the hydroxyapatite particles may be fixed to the constituent fiber surface of the base nonwoven fabric by being left at room temperature, or by cooling the base nonwoven fabric and the hydroxyapatite particles using an appropriate cooling means depending on needs, for example, by blowing a cooling air from the upper portion of the conveyor. As a method of heating the hydroxyapatite particles, for example, it is possible to exemplify a method of adding the hydroxyapatite particles into a heat-resisting container and heating the hydroxyapatite particles using an oven, or a method of placing the hydroxyapatite particles on a heat-resisting conveyor and continuously heating the hydroxyapatite particles using a heater provided on the upper portion of the conveyor while the conveyor is moved, or the like. As a method of heating the hydroxyapatite particles, an arbitrary heating method can be used as long as the method is a method capable of heating the whole hydroxyapatite particles. However, regarding a heating temperature at this time, it is necessary to heat the hydroxyapatite particles to a temperature higher than a melting point of a thermoplastic resin having the lowest melting point among the thermoplastic resins constituting the surfaces of the constituent fibers of the base nonwoven fabric.

### <Pre-treatment Step>

Here, as a pre-treatment step, it is preferable to wash (for example, wash with alcohol) the base nonwoven fabric according to the embodiment of the invention and to remove an oil solution, dirt, or the like attached to the nonwoven fabric in the course of the manufacturing of the base nonwoven fabric before the hydroxyapatite particles are brought into contact with the base nonwoven fabric. When the base nonwoven fabric itself is washed once, it is possible to obtain a functional nonwoven fabric excellent in hygiene, and as described later, it is possible to obtain a functional nonwoven fabric which is more suitable for application for oral use in which hygiene is considered to be important.

### <Post-treatment Step>

As a post-treatment step, it is also possible to sterilize the functional nonwoven fabric according to the embodiment of the invention. When the functional nonwoven fabric according to the embodiment of the invention is sterilized, it is possible to obtain a functional nonwoven fabric excellent in hygiene, and as described later, to obtain a functional nonwoven fabric which is more suitable for application for oral use in which hygiene is considered to be important. As a sterilization method, it is also possible to use any methods which are generally considered, and for example, ethylene oxide gas (EOG), electron beam irradiation, γ-ray irradiation, UV sterilization, autoclave, alcohol sterilization, plasma sterilization, and the like are exemplified. In the sterilization, the functional nonwoven fabric itself may be used or a product after packaging may be used. In a case where a perforated packaging material is used, a method of EOG, or autoclave can be employed, and a sterilization method using electron beam irradiation, γ-ray irradiation, or UV sterilization can be used by selecting a material even in the case of using a non-porous packaging material.

### <<Application Use of Functional Nonwoven Fabric>>

Next, the application use of the functional nonwoven fabric according to the embodiment of the invention will be described. Here, the functional nonwoven fabric according to the embodiment of the invention is suitable for, particularly, application for oral use. Hereinafter, a relation between a problem relating to general oral environment and the functional nonwoven fabric according to the embodiment of the invention as a solving means of the problem will be described in detail, and then other preferred application use will be described. Incidentally, the application use of the functional nonwoven fabric is not limited to application for human beings, and can be used for animals, and further can be widely used regardless of living organisms or non-living organisms.

### <For Oral Use>

A plaque present in the oral cavity is a membrane which is thin, colorless, and sticky and is almost invisible. This membrane is continuously formed not only in a large portion on the surfaces of teeth but also in the back of the tongue, a palate, and a mucous membrane. The plaque is composed by a microbe such as a bacterium, saliva, protein, and a residue of food.

Here, an etiologically main pathogen causing a carious tooth is considered to be a streptococcus mutans {gram (+) bacteria Streptococcus mutans} that is a gram-positive bacterium. The hard tissue (enamel) of the tooth becomes brittle due to the action of the streptococcus mutans and thus a hole (cavity) of the carious tooth is easily formed.

A principal cause of a periodontal disease is a plaque due to bacteria. When the plaque present in the oral cavity is not removed, the plaque is hardened and accumulated on the surface or the like of the tooth, and thus the periodontal disease proceeds. Moreover, in the periodontitis that is a stage where the periodontal disease is most advanced, a support tissue surrounding the tooth is destroyed; meanwhile, a pocket in which plaques due to a larger number of bacterium are accumulated is formed in a gingiva so that the tooth is lost.

In this way, the plaques are principal causes of the start and proceed of the carious tooth and the periodontal disease that are two most general oral diseases. Therefore, in order to improve the environment in the oral cavity, it is necessary to efficiently remove or reduce a harmful microbe and protein in the oral cavity and to suppress the formation of plaques in the oral cavity.

For the purpose of the improvement of the environment in the oral cavity, there is a case where sterilization is performed by using a mouthwash or the like obtained by mixing components having a strong sterilization effect and then contacting a bactericidal agent and bacteria, which are present in the oral cavity, in the oral cavity. However, generally, the component having a bacteria-killing capability has a strong stimulus property, and thus this component damages a sensitive mucous membrane in the oral cavity or necessary bacteria in the oral cavity. Moreover, there is a disadvantage that the relevant component has strong stimulus with respect to children or the elderly who have a brittle mucous membrane.

Here, hydroxyapatite that is one kind of calcium phosphate is a ceramic of a main component for a tooth or a bone, and as described above, since the hydroxyapatite has excellent biological compatibility, protein adsorption property, ion-exchange capacity, or the like, the hydroxyapatite is used as a compounding agent for a biomaterial or a dentifrice. In particular, since the hydroxyapatite has an adsorption action to bacteria, when the hydroxyapatite is mixed in a stomatological preparation (for example, a dentifrice), it is considered that the hydroxyapatite does not damage a sensitive mucous membrane in the oral cavity or the like and can adsorb a plaque in the oral cavity so as to remove bacterial plaque. As the stomatological preparation mixed with the hydroxyapatite having such an effect, it is possible to exemplify a dentifrice, a mouthwash, a chewing gum, or the like which is mixed with the hydroxyapatite.

In order to remove the plaque in the oral cavity by using an adsorption force of the hydroxyapatite, it is necessary to allow a microbe, protein, or the like to be adsorbed by hydroxyapatite particles and then to reliably remove such hydroxyapatite particles from the oral cavity. However, for example, in the case of using a dentifrice mixed with hydroxyapatite, there is a problem in that the hydroxyapatite adsorbing a plaque remains in the oral cavity even after washing by using the dentifrice and thus the percentage of the removal of a microbe or protein is decreased. The dentifrice is generally used together with a toothbrush. However, in a method of performing physical scraping by a toothbrush, it is difficult to allow the hydroxyapatite to come in sufficient contact with the plaque or oral tissue and a microbe adsorption effect is difficult to be exerted. For this reason, there is a problem in that it is necessary to mix a large amount of hydroxyapatite in order to improve the environment in the oral cavity.

A nonwoven fabric or a brush using a microfiber is known as a product for improving the environment in the oral cavity such as a tooth or tongue coat. According to this, it is possible to remove a microbe or protein from the oral cavity by physical scraping.

However, since a microbe or protein is adsorbed to the inside of the oral cavity, the removal of the microbe by physical scraping is not sufficient and they may remain in the oral cavity. If scraping is intended to be sufficiently performed, a mucous membrane or the like in the oral cavity is stimulated and damaged. As a result, there is a disadvantage that the improvement of the environment in the oral cavity is not sufficient.

On the other hand, according to the functional nonwoven fabric relevant to the embodiment of the invention, since the functional nonwoven fabric has good feeling upon use, such as hand feeling (softness) or texture, and has a state in which the particles are appropriately fixed to the base nonwoven fabric, the characteristics of hydroxyapatite can be stably exerted. That is, in the functional nonwoven fabric according to the embodiment of the invention, the base nonwoven fabric itself is soft. For this reason, in a case where a three-dimensional surface such as a tooth is wiped off, the functional nonwoven fabric can follow the unevenness thereof and has an excellent effect of physically scraping a microbe or protein together with an adsorbing effect of the hydroxyapatite fixed to the fiber. Since a mucous membrane or the like in the oral cavity is less likely to be stimulated even when being contacted with the functional nonwoven fabric according to the embodiment of the invention, the functional nonwoven fabric can be particularly preferably used as a functional nonwoven fabric for oral use (for example, a sheet for tongue coat or a dental sheet).

The application use of the functional nonwoven fabric according to the embodiment of the invention is particularly preferably used for oral use in this way. Moreover, the functional nonwoven fabric according to the embodiment of the invention can be preferably used as a functional nonwoven fabric product which can effectively adsorb dirt, a microbe, or the like under the environment where water cannot be used, such as a dirt adsorbing sheet or the like for nursing care, travelling (for example, application use in a vehicle), amenity, hygiene, or emergency supplies. Since the functional nonwoven fabric can efficiently remove a microbe or protein in the oral cavity in a short time, the functional nonwoven fabric can be preferably used as an oral environment conditioning material (caries prevention, periodontal disease prevention, breath odor suppression, or the like) for animals which do not like tooth-brushing.

### <Other Application Use>

The functional nonwoven fabric according to the embodiment of the invention can be preferably used, for example, as a cosmetic base material that has a low physical stimulus property to skin, such as a pollen-removing sheet, a body-washing sheet, a makeup-removing sheet, a facial mask, or a sheet for babies. Moreover, the functional nonwoven fabric not only can remove pollen, dirt due to lipid or protein, a microbe, or the like, but also can prevent various allergy diseases.

In a case where a wound region or the like is covered with the functional nonwoven fabric according to the embodiment of the invention, since an infection causative microbe is adsorbed by hydroxyapatite fixed to the base nonwoven fabric and the functional nonwoven fabric adsorbing the causative microbe can be simply replaced, the functional nonwoven fabric can be used for sterilization of the infection causative microbe in the wound region.

In addition, when various medical agents, microorganisms, or physiologically active substances are adsorbed in advance to hydroxyapatite fixed to the functional nonwoven fabric according to the embodiment of the invention, it is possible to impart a new function to the functional nonwoven fabric. For example, the functional nonwoven fabric can be used as a carrier for an immobilized enzyme, or can be also employed as a carrier for a diagnostic pharmaceutical in an immunoassay method by allowing a specific antibody to be adsorbed by the functional nonwoven fabric. The functional nonwoven fabric can be employed as a biocatalyst by being used as a carrier for a microorganism or the like, or in a case where the functional nonwoven fabric is used as a wound dressing base material or a cosmetic base material, since hydroxyapatite can exert an effect of moderately releasing a medical agent, the functional nonwoven fabric can also stably maintain an effect of a medical agent to be effective for a long period of time.

The functional nonwoven fabric according to the embodiment of the invention can be filled and infiltrated in advance with a fluid in various application uses. In particular, when a fluid obtained by dispersing hydroxyapatite particles is infiltrated into the functional nonwoven fabric according to the embodiment of the invention, it is possible to further enhance an effect of removing protein dirt or a microbe from the surface of a target for which the functional nonwoven fabric is used. In addition, in accordance with an application use, water, a solvent such as alcohol, various cleansing components, a perfume material, a wetting agent, a moisturizing ingredient, or a preserving agent can be used, and in the case of oral use, a sweetening agent, an algefacient, or the like can be used.

### <<Usage Type of Functional Nonwoven Fabric>>

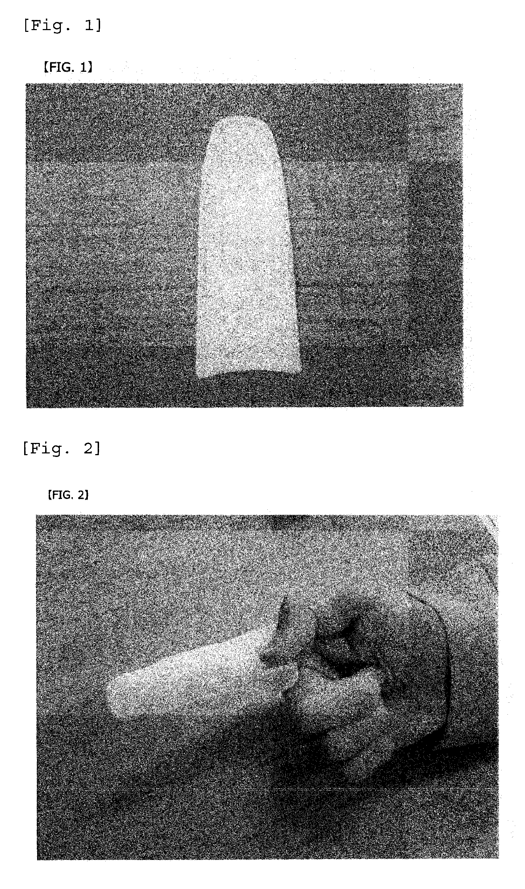

The functional nonwoven fabric according to the embodiment of the invention can be used in a sheet form as it is, can be used by being cut into a size in which the functional nonwoven fabric is easily used to form a functional nonwoven fabric sheet and, for example, winding the sheet on a finger, or can be used after being processed in various shapes. For example, as illustrated in Fig. 1, the functional nonwoven fabric according to the embodiment of the invention is processed into a shape of a fingerstall and then the fingerstall-shaped functional nonwoven fabric is fitted to a finger for use, as illustrated in Fig. 2. According to this, this functional nonwoven fabric is preferably used particularly in the case of using for a small space such as the inside of the oral cavity (the application is not limited to the oral use for humans, and for example, can also be made for the oral use for pets and the like).

### Examples

### <<Preparation of Functional Nonwoven Fabric>>

A functional nonwoven fabric was prepared according to the following procedures.
1. Preparation of Base Nonwoven Fabric
2. Fixation of Hydroxyapatite Particles to Base Nonwoven Fabric

Hereinafter, description on each procedure will be made sequentially.

### <1. Preparation of Base Nonwoven Fabric>

### (Base Nonwoven Fabric A)

70% by mass of a core-sheath type composite fiber (a fineness of 6.6 dtex and a fiber length of 102 mm) composed of polyethylene (a melting point of 130°C)/polypropylene (a melting point of 160°C), 25% by mass of a core-sheath type composite fiber (a fineness of 2.2 dtex and a fiber length of 51 mm) composed of polyester (a melting point of 255°C)/low melting point polyester (a melting point of 245°C), and 5% by mass of a core-sheath type composite fiber (a fineness of 3.3 dtex and a fiber length of 64 mm) composed of an ethylene-ethylene-vinyl acetate copolymer (a melting point of 110°C)/polypropylene (a melting point of 160°C) were mixed and then a web a having an area density of 30 g/m² was prepared by a carding machine.

Subsequently, 30% by mass of a core-sheath type composite fiber (a fineness of 20 dtex and a fiber length of 102 mm) composed of polyethylene (a melting point of 130°C)/polypropylene (a melting point of 160°C), 65% by mass of a core-sheath type composite fiber (a fineness of 6.6 dtex and a fiber length of 102 mm) composed of polyethylene (a melting point of 130°C)/polypropylene (a melting point of 160°C), and 5% by mass of a core-sheath type composite fiber (a fineness of 3.3 dtex and a fiber length of 64 mm) composed of ethylene-ethylene-vinyl acetate copolymer (a melting point of 110°C)/polypropylene (a melting point of 160°C) were mixed and then a web b having an area density of 50 g/m² was prepared by a carding machine.

Subsequently, these webs were superimposed and the needle punching treatment was performed on the web a side, thereby obtaining a base nonwoven fabric A having an area density 80 g/m², which was a double-layered nonwoven fabric.

### (Preparation of Base Nonwoven Fabric B)

70% by mass of a fiber (a fineness of 2.2 dtex and a fiber length of 51 mm) composed of polypropylene (a melting point of 160°C), 25% by mass of a rayon fiber (a fineness of 1.7 dtex and a fiber length of 40 mm), and 5% by mass of a core-sheath type composite fiber (a fineness of 1.7 dtex and a fiber length of 51 mm) composed of polyethylene (a melting point of 130°C)/polypropylene (a melting point of 160°C) were mixed and then a web c having an area density of 60 g/m² was prepared by a carding machine.

Subsequently, the hydroentangling treatment was performed on the web c, thereby obtaining a base nonwoven fabric B having an area density of 60 g/m².

### (Preparation of Base Nonwoven Fabric C)

60% by mass of a rayon fiber (a fineness 1.7 dtex and a fiber length of 40 mm) and 40% by mass of a core-sheath type composite fiber (a fineness of 1.7 dtex and a fiber length of 51 mm) composed of polyethylene (a melting point of 130°C)/polypropylene (a melting point of 160°C) were mixed and then a web d having an area density of 70 g/m² was prepared by a carding machine.

Subsequently, the hydroentangling treatment was performed on the web d, thereby obtaining a base nonwoven fabric C having an area density of 70 g/m².

### (Preparation of Base Nonwoven Fabric D)

80% by mass of a rayon fiber (a fineness of 3.3 dtex and a fiber length of 51 mm), 15% by mass of a fiber (a fineness of 2.2 dtex and a fiber length of 50 mm) composed of polypropylene (a melting point of 160°C), and 5% by mass of a core-sheath type composite fiber (a fineness of 1.7 dtex and a fiber length of 51 mm) composed of polyethylene (a melting point of 130°C)/polypropylene (a melting point of 160°C) were mixed and then a web e having an area density of 60 g/m² was prepared by a carding machine.

Subsequently, the hydroentangling treatment was performed on the web e, thereby obtaining a base nonwoven fabric D having an area density of 60 g/m².

### (Preparation of Base Nonwoven Fabric E)

A web f having an area density 30 g/m² was prepared using a split-type composite fiber (a fineness of 3.3 dtex and a fiber length of 51 mm) composed of polyester (a melting point of 255°C)/polypropylene (a melting point of 160°C) by a carding machine.

Subsequently, 80% by mass of a fiber (a fineness of 1.45 dtex and a fiber length of 38 mm) composed of polyester (a melting point of 255°C) and 20% by mass of a split-type composite fiber (a fineness of 3.3 dtex and a fiber length of 51 mm) composed of polyester (a melting point of 255°C)/polypropylene (a melting point of 160°C) were mixed and then a web g having an area density 30 g/m² was prepared by a carding machine.

Subsequently, these webs were superimposed and the hydroentangling treatment was performed on the web f side, thereby obtaining a base nonwoven fabric E having an area density 60 g/m², which was a double-layered nonwoven fabric and contained a polyester fiber and a polypropylene fiber of which cross-sectional shapes are wedge-shaped, the fibers being obtained by dividing the split-type composite fiber.

### (Preparation of Base Nonwoven Fabric F)

60% by mass of a fiber (a fineness of 1.5 dtex and a fiber length of 38 mm) composed of polyester (a melting point of 255°C), 20% by mass of a split-type composite fiber (a fineness of 3.3 dtex and a fiber length of 51 mm) composed of polyester (a melting point of 255°C)/polypropylene (a melting point of 160°C), and 20% by mass of a core-sheath type composite fiber (a fineness of 2.2 dtex and a fiber length of 51 mm) composed of polyester (a melting point of 255°C)/low melting point polyester (a melting point of 110°C) were mixed and then a web h having an area density 30 g/m² was prepared by a carding machine.

Subsequently, 80% by mass of a split-type composite fiber (a fineness of 3.3 dtex and a fiber length of 51 mm) composed of polyester (a melting point of 255°C)/polypropylene (a melting point of 160°C) and 20% by mass of a core-sheath type composite fiber (a fineness of 2.2 dtex and a fiber length of 51 mm) composed of polyester (a melting point of 255°C)/low melting point polyester (a melting point of 110°C) were mixed and then a web i having an area density 20 g/m² was prepared by a carding machine.

Subsequently, these webs were superimposed and the hydroentangling treatment was performed on the web h side, thereby obtaining a base nonwoven fabric F having an area density 50 g/m², which was a double-layered nonwoven fabric and contained a polyester fiber and a polypropylene fiber of which cross-sectional shapes are wedge-shaped, the fibers being obtained by dividing the split-type composite fiber.

### (Preparation of Base Nonwoven Fabric G)

70% by mass of a fiber (a fineness of 1.45 dtex and a fiber length of 38 mm) composed of polyester (a melting point of 255°C) and 30% by mass of a core-sheath type composite fiber (a fineness of 2.8 dtex and a fiber length of 51 mm) composed of an ethylene-vinyl alcohol copolymer (a melting point of 175°C)/polypropylene (a melting point of 160°C) were mixed and then a web m having an area density 60 g/m² was prepared by a carding machine.

Subsequently, the hydroentangling treatment was performed on the web m, thereby obtaining a base nonwoven fabric G having an area density of 70 g/m².

### <2. Fixation of Hydroxyapatite Particles to Base Nonwoven Fabric>

### (Examples 1 to 6)

The sintered hydroxyapatite particles having a substantially spherical shape (produced by SofSera Corporation, product name: micro-SHAp, product number: IHM-100P000, particle diameter: 4.0 µm) were heated to 220°C, and were blown to the base nonwoven fabrics A to F together with an airflow at 160°C, followed by being allowed to cool. Subsequently, the hydroxyapatite particles, which were entangled between the constituent fibers of the base nonwoven fabric and were not fixed to the fiber surface, were removed by air. Incidentally, the fixed amount of the hydroxyapatite particle was adjusted by the amount of the hydroxyapatite particle to be blown to the base nonwoven fabric. In this way, functional nonwoven fabrics according to Examples 1 to 6 in which the hydroxyapatite particles were fixed to the surfaces of the fibers obtained from the thermoplastic resin as presented in Table 1 were obtained.

### (Comparative Example 1)

A functional nonwoven fabric according to Comparative Example 1 as presented in Table 1 was obtained in the same manufacturing method in Example 1, except that the unsintered hydroxyapatite particles having a substantially spherical shape (produced by SofSera Corporation, product name: unsintered micro-SHAp, particle diameter: 4.0 µm) were used.

### (Comparative Example 2)

A dispersion liquid obtained by dispersing the sintered hydroxyapatite particles having a substantially spherical shape (produced by SofSera Corporation, product name: micro-SHAp, product number: IHM-100P000, particle diameter: 4.0 µm) was heated to 95°C and the base nonwoven fabric G was immersed in the dispersion liquid for 10 minutes. Subsequently, the hydroxyapatite particles, which were entangled between the constituent fibers of the base nonwoven fabric and were not fixed to the fiber surface, were removed by flowing water. In this way, a functional nonwoven fabric according to Comparative Example 2 in which the hydroxyapatite particles were fixed to the surfaces of the fibers obtained from the thermoplastic resin as presented in Table 1 was obtained.

### (Comparative Example 3)

After a dispersion liquid obtained by dispersing the sintered hydroxyapatite particles having a substantially spherical shape (produced by SofSera Corporation, product name: micro-SHAp, product number: IHM-100P000, particle diameter: 4.0 µm) was immersed in the base nonwoven fabric A, the whole base nonwoven fabric A was heated for 10 minutes at a temperature (140°C) at which a portion of the base nonwoven fabric A is melted in a drying machine, followed by being allowed to cool. Therefore, a nonwoven fabric in which part of the hydroxyapatite particles were fixed to the fibers by thermal fusion bonding was prepared. The hydroxyapatite particles, which were entangled between the constituent fibers of the base nonwoven fabric and were not fixed to the fiber surface, were removed by air. In this way, a functional nonwoven fabric according to Comparative Example 3 in which the hydroxyapatite particles were fixed to the surfaces of the fibers obtained from the thermoplastic resin as presented in Table 1 was obtained.

### (Comparative Example 4)

A functional nonwoven fabric according to Comparative Example 4 as presented in Table 1 was obtained in the same manufacturing method in Example 1, except that the unsintered hydroxyapatite particles having a non-spherical shape (produced by Taihei Chemical Industrial Co., Ltd., product name: Hydroxyapatite, particle diameter: 5.0 µm) were used.

### <<Evaluation Test>>

Next, various evaluation tests were carried out by using the prepared functional nonwoven fabrics according to Examples 1 to 6 and Comparative Examples 1 to 4.

### <Test 1> XRD Spectrum of Particles

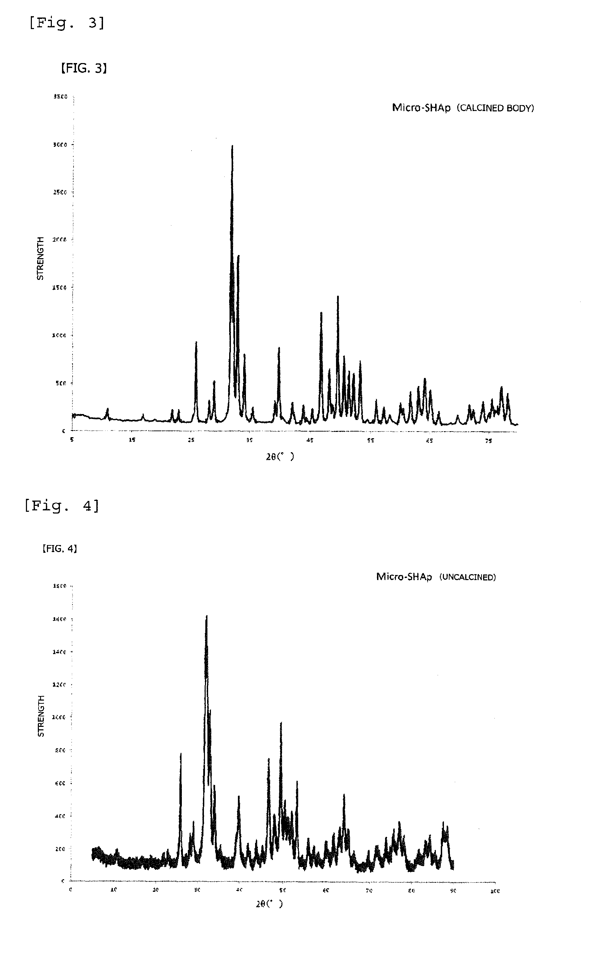

The sintered hydroxyapatite particles used in Examples 1 to 6 and Comparative Examples 2 and 3 and the unsintered hydroxyapatite particles used in Comparative Example 1 were analyzed under the following conditions by using XRD. The results thereof are presented in Fig. 3 and Fig. 4.

### (Analysis Conditions)

The measurement was performed by an X-ray analyzing device Mini Flex/HCM (manufactured by Rigaku Corporation). The measurement conditions are as follows.
· Target: CuKα
· Tube voltage: 30 kV
· Tube current: 15 mA
· Scanning range: 5° to 90°
· Scanning speed: 1.000°/min
· Scattering slit: 4.2°
· Receiving slit: 0.3 mm

As illustrated in Fig. 3, since the half widths of the hydroxyapatite particles used in Examples 1 to 6 and Comparative Examples 2 and 3 at a peak of 2θ = 31.8 (d = 2.814) were 0.2, it was confirmed that the hydroxyapatite particles were sintered. On the other hand, as illustrated in Fig. 4, since the hydroxyapatite particles used in Comparative Example 1 had poor peak separation and the half width at a peak of 2θ = 31.8 (d = 2.814) was, although not exactly calculated, about 1.2, it was confirmed that the hydroxyapatite particles were unsintered.

### <Test 2> Measurement of Aspect Ratio

Regarding Example 1, the aspect ratio of each hydroxyapatite particle fixed to the constituent fibers of the base nonwoven fabric was 1.15. The measuring method of the aspect ratio was according to the measuring method (1) described above. Similarly, the aspect ratio of each hydroxyapatite particle supported by the functional nonwoven fabrics according to Examples 2 to 6 and Comparative Examples 1 to 3 was measured to be 1.45 or less. Similarly, regarding Comparative Example 4, the aspect ratio of each hydroxyapatite particle fixed to the constituent fibers of the base nonwoven fabric was measured, and as a result, an average aspect ratio exceeded 1.45.

### <Test 3> Measurement of Particle Diameter

Regarding Example 1, the particle diameter of each hydroxyapatite particle fixed to the constituent fibers of the base nonwoven fabric was 4.42 µm. The measuring method of the particle diameter was according to the measuring method (2) described above. Incidentally, the particle diameter of each hydroxyapatite particle supported by the functional nonwoven fabrics according to Examples 2 to 6 and Comparative Examples 1 to 4 was measured to be 0.1 µm or more but 10 µm or less.

### <Test 4> Shearing Rigidity Test

Regarding the functional nonwoven fabrics of Examples 1 to 6 and Comparative Examples 1 to 4, the shearing rigidity G was measured according to the measuring method (3). The results thereof are presented in Table 2.

### <Test 5> Hand feeling Sensory Test

Regarding the functional nonwoven fabrics of Examples 1 to 6 and Comparative Examples 1 to 4, hand feeling when the functional nonwoven fabric touched the skin was evaluated by 10 monitoring persons. The evaluation was conducted in such a manner that each monitoring person selected and answered whether the hand feeling of the functional nonwoven fabric is soft or hard and then the evaluation was conducted by the number of persons who answered "soft." The results thereof are presented in Table 2.

### <Test 6> Touch Sensory Test

In order to confirm a difference in texture due to the difference of the hydroxyapatite particle, regarding the functional nonwoven fabrics of Example 1, Comparative Example 1, and Comparative Example 4, texture when the functional nonwoven fabric touched the skin was evaluated by 10 monitoring persons. The evaluation was conducted according to the following evaluation criteria and in 5 grades, and then scoring was performed. The results thereof are presented in Table 3.
5 point: good (texture is good)
4 point: slightly good (texture is good)
3 point: neither good nor poor (texture is neither good nor poor)
2 point: slightly poor (texture is slightly poor)
1 point: poor (texture is poor)

### <Test 7> Static Friction Test

In order to confirm a relation between the coefficient of static friction and the texture due to the difference between the sintered hydroxyapatite particle and the unsintered hydroxyapatite particle, regarding the functional nonwoven fabrics of Example 1 and Comparative Example 1, a coefficient of static friction on the surface of the functional nonwoven fabric was measured according to the measuring method (4). The results thereof are presented in Table 4.

[Table 1]

**[Table 1]**

| | Base nonwoven fabric | Fixed amount [g/m²] | Hydroxyapatite particle |
|---|---|---|---|
| Example 1 | Base nonwoven fabric A | 1 | micro-SHAp produced by SofSera Corporation |
| Example 2 | Base nonwoven fabric B | 1 | micro-SHAp produced by SofSera Corporation |
| Example 3 | Base nonwoven fabric c | 2 | micro-SHAp produced by SofSera Corporation |
| Example 4 | Base nonwoven fabric D | 1 | micro-SHAp produced by SofSera Corporation |
| Example 5 | Base nonwoven fabric E | 1 | micro-SHAp produced by SofSera Corporation |
| Example 6 | Base nonwoven fabric F | 1 | micro-SHAp produced by SofSera Corporation |
| Comparative Example 1 | Base nonwoven fabric A | 1 | unsintered micro-SHAp produced by SofSera Corporation |
| Comparative Example 2 | Base nonwoven fabric G | 1 | micro-SHAp produced by SofSera Corporation |
| Comparative Example 3 | Base nonwoven fabric A | 1 | micro-SHAp produced by SofSera Corporation |
| Comparative Example 4 | Base nonwoven fabric A | 1 | Hydroxyapatite produced by Taihei Chemical Industrial Co., Ltd. |

[Table 2]

**[Table 2]**

| | Average value of shearing rigidity G [gf/cm/degree] | Drape sensory evaluation Number of persons who answered "soft" [person] |
|---|---|---|
| Example 1 | 5.7 | 10 |
| Example 2 | 6.3 | 10 |
| Example 3 | 6.2 | 10 |
| Example 4 | 3.7 | 10 |
| Example 5 | 5.6 | 10 |
| Example 6 | 7.4 | 8 |
| Comparative Example 1 | 5.7 | 10 |
| Comparative Example 2 | 9.3 | 2 |
| Comparative Example 3 | 13.5 | 0 |
| Comparative Example 4 | 5.7 | 10 |

[Table 3]

**[Table 3]**

| | Example 1 | Comparative Example 1 | Comparative Example 4 |
|---|---|---|---|
| 5 point | 1 person | 0 person | 0 person |
| 4 point | 6 persons | 2 persons | 0 person |
| 3 point | 3 persons | 6 persons | 5 persons |
| 2 point | 0 person | 2 persons | 4 persons |
| 1 point | 0 person | 0 person | 1 person |
| Total | 38 | 30 | 24 |

[Table 4]

**[Table 4]**

| | Coefficient of static friction · Average value |
|---|---|
| Example 1 | 0.2 |
| Comparative Example 1 | 0.32 |

### «Evaluation Result»

From Table 2, the functional nonwoven fabrics according to Comparative Examples 2 and 3 had the shearing rigidity value G exceeding 8.5 gf/cm/degree. In a method including a step of heating the whole base nonwoven fabric, particularly, a method including a step of heating the whole base nonwoven fabric at a temperature at which at least a portion of the fiber composing the base nonwoven fabric is fused (Comparative Example 3) as the method for manufacturing the functional nonwoven fabric according to Comparative Examples 2 and 3, that is, the method of fixing hydroxyapatite, it was considered that the fibers were fused, and as a result, softness (soft hand feeling) of the base nonwoven fabric was lost.

According to the hand feeling (softness) sensory test presented in Table 2, it was determined that the functional nonwoven fabrics according to Examples 1 to 6 having a shearing rigidity value G of 8.5 gf/cm/degree or less were excellent in hand feeling. On the other hand, regarding Comparative Example 2 (9.3 gf/cm/degree) and Comparative Example 3 (13.5 gf/cm/degree) having a shearing rigidity value G exceeding 8.5 gf/cm/degree, it was determined that they were inferior in hand feeling. In this way, in a case where the shearing rigidity value G is high, it is considered that the functional nonwoven fabric is not excellent in hand feeling and the high shearing rigidity value causes adverse effects on feeling upon use, for example, when the functional nonwoven fabric is used for skin, a mucous membrane, or the like of humans.

For the results in Table 3, it is found that the functional nonwoven fabric according to Example 1 to which the sintered hydroxyapatite particles are fixed is excellent in texture as compared with the functional nonwoven fabric according to Comparative Example 1 and the functional nonwoven fabric according to Comparative Example 4 to which the unsintered hydroxyapatite particles are fixed. Similarly, from the results in Table 4, it is found that, when the sintered hydroxyapatite particles are fixed, the coefficient of static friction of the functional nonwoven fabric is decreased as compared with the functional nonwoven fabric to which the unsintered hydroxyapatite particles are fixed. The reason for this is presumed that, in the functional nonwoven fabric according to Comparative Example 1, the unsintered hydroxyapatite is collapsed by a load during a friction test, the particles are likely to be left out from the functional nonwoven fabric, and thus the particles act like polishing components, and as a result, a friction coefficient is increased. Therefore, in the combination with the results in Table 3, it is found that, in a case where the functional nonwoven fabrics according to Examples of the invention are used for, particularly, a portion vulnerable to friction such as skin or a mucous membrane of humans, for example, for the wiping-off application, it is preferable to use the functional nonwoven fabric of Example 1 in which a target to be wiped off is less likely to be damaged and which is excellent in texture, that is, the functional nonwoven fabric using the sintered hydroxyapatite particles is preferably used. Incidentally, here, from the results in Table 3, when Comparative Example 1 using substantially spherical hydroxyapatite particles is compared with Comparative Example 4 using non-spherical hydroxyapatite particles, Comparative Example 4 is inferior in texture as compared with Comparative Example 1. Therefore, it is found that the hydroxyapatite particles are preferably substantially spherical.

## Claims

1. A functional nonwoven fabric in which sintered hydroxyapatite particles are fixed by thermal fusion bonding to a surface of a nonwoven fabric containing fibers, at least surfaces of the fibers being mainly composed of a thermoplastic resin,
wherein a shearing rigidity of the functional nonwoven fabric is 8.5 gf/cm/degree or less.

2. The functional nonwoven fabric according to claim 1, wherein an aspect ratio of the sintered hydroxyapatite particle is 1.45 or less.

3. The functional nonwoven fabric according to claim 1 or 2, wherein a particle diameter of the sintered hydroxyapatite particle is 0.1 µm or more but 10 µm or less.

4. The functional nonwoven fabric according to any one of claims 1 to 3, which is for oral use.

5. A method for manufacturing a functional nonwoven fabric, the method comprising blowing an airflow containing sintered hydroxyapatite particles, which have been heated to a temperature higher than a melting point of a thermoplastic resin, to a surface of a nonwoven fabric containing fibers, at least surfaces of the fibers being mainly composed of the thermoplastic resin, so that the sintered hydroxyapatite particles are fixed by thermal fusion bonding to the surface of the nonwoven fabric.

6. The method for manufacturing a functional nonwoven fabric according to claim 5, wherein an aspect ratio of the sintered hydroxyapatite particle is 1.45 or less.

7. The method for manufacturing a functional nonwoven fabric according to claim 5 or 6, wherein a particle diameter of the sintered hydroxyapatite particle before being blown to the surface of the nonwoven fabric is 0.1 µm or more but 10 µm or less.

8. The method for manufacturing a functional nonwoven fabric according to any one of claims 5 to 7, wherein the functional nonwoven fabric is for oral use.
